# EUROPEAN PATENT APPLICATION

(11) **EP 4 353 183 A1**
(43) Date of publication of application: **17.04.2024**
(21) Application number: 22815598.2
(22) Date of filing: 02.03.2022
(51) Int. Cl.: A61B 34/35, G16H 40/67, H04N 7/15, G06F 3/01

(54) **INFORMATION PROCESSING DEVICE, INFORMATION PROCESSING SYSTEM, INFORMATION PROCESSING METHOD, AND PROGRAM**

(30) Priority: 03.06.2021 JP 2021093551
(71) Applicant: Sony Group Corporation, Tokyo 108-0075 (JP)
(72) Inventor: KATSUKI, Shinji, Tokyo 108-0075 (JP); MATSUURA, Kana, Tokyo 108-0075 (JP); KOBAYASHI, Motoaki, Tokyo 108-0075 (JP); MATSUURA, Hiromitsu, Tokyo 108-0075 (JP)
(74) Representative: D Young & Co LLP
(86) International application number: PCT/JP2022/008833
(87) International publication number: WO 2022/254840

(57) **Abstract**

To improve privacy protection. An information processing apparatus according to an embodiment includes: an input unit (22) that acquires information including an operation by a user on a medical device on a computer network; an acquisition unit (21) that acquires operation information for remotely operating the medical device on the basis of the operation input to the input unit; a generation unit (21) that generates operation data for remotely operating the medical device using the operation information; and a transmission unit (21) that transmits the operation data to the computer network, in which the operation data does not include personal information of the user.

## Description

### Field

The present disclosure relates to an information processing apparatus, an information processing system, an information processing method, and a program.

### Background

In recent years, in the medical field such as surgery, medical systems that enable remote operation of medical devices such as an endoscope and an electric scalpel have been developed. For example, Patent Literature 1 below proposes a method of operating a medical device by recognizing a face, a line-of-sight, and a voice of a medical staff such as a doctor and a nurse.

### Citation List

### Patent Literature

Patent Literature 1: JP 2017-512554 A

### Summary

### Technical Problem

However, in a case where a medical device and an input device for remotely operating the medical device are connected via a network, such as a local area network (LAN), accessible by a person other than a staff involved in diagnosis, surgery, or the like, or by a device other than a medical device used for diagnosis, surgery, or the like, for example, there is a problem in that when information or the like for remotely operating the medical device is transmitted to the network as it is, information regarding privacy of the staff who operates the medical device or the patient may leak to the outside, and privacy of a person involved in the surgery or the diagnosis may be impaired.

Therefore, the present disclosure proposes an information processing apparatus, an information processing system, an information processing method, and a program capable of improving privacy protection.

### Solution to Problem

An information processing apparatus according to the present disclosure includes: an input unit configured to acquire information including an operation by a user on a medical device on a computer network; an acquisition unit configured to acquire operation information for remotely operating the medical device on a basis of the operation input to the input unit; a generation unit configured to generate operation data for remotely operating the medical device using the operation information; and a transmission unit configured to transmit the operation data to the computer network, wherein the operation data does not include personal information of the user.

### Brief Description of Drawings

FIG. 1 is a block diagram illustrating a schematic configuration example of a surgical integrated imaging system according to an embodiment of the present disclosure.
FIG. 2 is a block diagram illustrating a more detailed schematic configuration example of the surgical integrated imaging system according to the embodiment of the present disclosure.
FIG. 3 is a diagram illustrating an example of a positional relationship between a display device and a surgical staff according to the embodiment of the present disclosure.
FIG. 4 is a diagram illustrating an example of a correspondence relationship between a face gesture and operation information according to the embodiment of the present disclosure.
FIG. 5 is a flowchart illustrating a series of flows of an operation example of remotely operating an endoscope by a face gesture according to the embodiment of the present disclosure.
FIG. 6 is a diagram illustrating an example of a user interface according to the embodiment of the present disclosure.
FIG. 7 is a diagram illustrating an example of a correspondence relationship between a line-of-sight action and operation information according to the embodiment of the present disclosure.
FIG. 8 is a flowchart illustrating a series of flows in an operation example of remotely operating an endoscope 31 by a line-of-sight action according to the embodiment of the present disclosure.
FIG. 9 is a flowchart illustrating an operation example of remotely operating a focus of an endoscope on the basis of a combination of a line-of-sight and image recognition according to the embodiment of the present disclosure.
FIG. 10 is a flowchart illustrating an operation example of remotely operating an endoscope on the basis of a combination of a line-of-sight and voice according to the embodiment of the present disclosure.
FIG. 11 is a flowchart illustrating an operation example of remotely operating an endoscope with a specific operation sound as a trigger according to the embodiment of the present disclosure.
FIG. 12 is a diagram illustrating an example of a screen displayed on a display unit according to the embodiment of the present disclosure.
FIG. 13 is a diagram illustrating an example of a command history confirmation screen according to the embodiment of the present disclosure.
FIG. 14 is a block diagram illustrating a schematic configuration example of a surgical integrated imaging system according to a modification of the embodiment of the present disclosure.
FIG. 15 is a hardware configuration diagram illustrating an example of a computer that implements functions according to the embodiment.

### Description of Embodiments

Hereinafter, preferred embodiments of the present disclosure will be described in detail with reference to the accompanying drawings. Note that, in the present specification and the drawings, components having substantially the same functional configuration are denoted by the same reference numerals, and redundant description is omitted.

Note that the description will be given in the following order.
0. Introduction
1. One Embodiment
1.1 System Configuration Example
1.2 Operation Example
1.2.1 Case of Remote Operation by Face Gesture
1.2.2 Case of Remote Operation on the basis of Line-of-sight
1.2.3 Case of Remote Operation by Combination of Line-of-sight and Image Recognition
1.2.4 Case of Remote Operation by Combination of Line-of-sight and Voice
1.2.5 Case of Remote Operation with Specific Operation Sound as Trigger
1.2.6 Case of Remote Operation of Other Electronic Device
1.3 Display of Transmission Command History
1.4 Modification of System Configuration
1.5 Summary

### 2. Hardware Configuration

### 0. Introduction

In recent years, surgical systems and medical systems that communicably connect medical devices, input devices, display devices, and the like via a computer network such as a LAN, a mobile communication system, or the Internet have been developed. On the other hand, in a surgical system or a medical system that enables remote operation of a medical device, a system that enables voice input or gesture input of operation information in consideration of convenience of operation input is conceivable.

However, unlike a dedicated line, the computer network is a network that can be directly accessed by a person, a device, or the like (hereinafter, also referred to as other devices) not directly related to surgery or diagnosis. Therefore, in a case where a computer network is applied to a surgical system or a medical system that enables remote operation of a medical device, if operation information input to an input device is transmitted as it is to the computer network, information regarding privacy of a staff who operates the medical device and a patient can be received by another device connected to the computer network, and there is a possibility that privacy of a person involved in the surgery or diagnosis is impaired.

Therefore, in the following embodiments, an information processing apparatus, an information processing method, and a program capable of improving the protection of privacy of a person involved in a specific surgery or diagnosis are proposed.

### 1. One Embodiment

Hereinafter, an information processing apparatus, an information processing system, an information processing method, and a program according to an embodiment of the present disclosure will be described in detail with reference to the drawings.

### 1.1 System Configuration Example

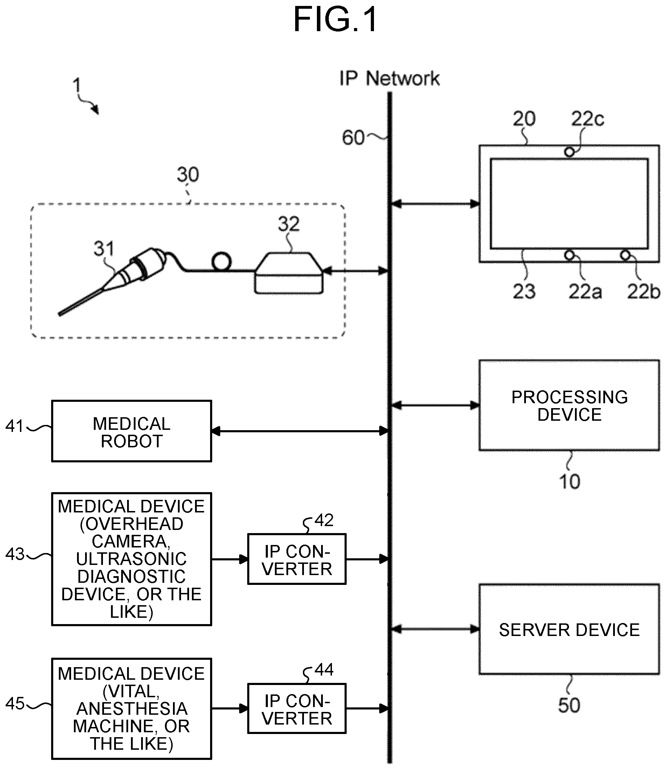

FIG. 1 is a block diagram illustrating a schematic configuration example of a surgical integrated imaging system according to the present embodiment. As illustrated in FIG. 1, a surgical integrated imaging system 1 has a configuration in which a processing device 10, a display device 20, and an endoscope system 30 are connected to each other via an Internet Protocol (IP) network 60.

The IP network 60 is an example of a computer network according to the present embodiment. The IP network 60 may be connected to a local network in the hospital or an external network (including a cloud network or the like) via some kind of gate to achieve cooperation with other devices, acquisition of information of preoperative data from a network, and exchange of information such as navigation used for surgery and connections for AI analysis, AI control, and the like. The IP network 60 is not limited to a communication network conforming to a communication protocol such as Transmission Control Protocol/Internet Protocol (TCP/IP) or User Datagram Protocol/Internet Protocol (UDP/IP), and may be various networks such as a mobile communication system (4th Generation Mobile Communication System (4G), 4G-Long Term Evolution (LTE), 5th Generation Mobile Communication System (5G)), and the like are included). Note that the IP network 60 is preferably a network capable of transmitting video data with a resolution of 4K, 8K, or higher.

In addition, as a part of the surgical integrated imaging system 1, the IP network 60 may be connected with a medical robot 41 that can be remotely operated, a medical device 43 such as an overhead camera or an ultrasonic diagnostic device for imaging the inside of the operating room or the operating field, a medical device 45 such as a device for measuring a vital such as a pulse, a heart rate, a respiration (frequency), a blood pressure, a body temperature, or the like, an anesthesia machine, a server device 50, and the like. Note that the medical devices 43 and 45 may be connected to the IP network 60 via IP converters 42 and 44 for transmitting the acquired data via the IP network 60, respectively.

The endoscope system 30 includes, for example, an endoscope 31 and a camera control unit (CCU) 32. A video imaged by the endoscope 31 (hereinafter, referred to as imaging data) is converted by the CCU 32 into a format that can be transmitted and received on the IP network 60, and is transmitted to the processing device 10 via the IP network 60. The endoscope 31 may be capable of imaging at a resolution of 4K, 8K, or higher. In addition, the CCU 32 generates a drive signal for driving the endoscope 31 on the basis of control data received from the processing device 10 via the IP network 60, thereby controlling the focus, magnification, and the like of the endoscope 31. At this time, in a case where the endoscope 31 is supported by a robot arm or the like for controlling its position and posture, the CCU 32 may control the robot arm in accordance with the control data received from the processing device 10 via the IP network 60.

The display device 20 (also referred to as smart medical display) will be described in detail later. However, for example, the display device 20 is provided with a display unit 23, and a video acquired by the endoscope system 30, the medical device 43, or the like and processed by the processing device 10 is displayed to a surgical staff such as a primary surgeon, a secondary surgeon, or a nurse. Note that the display unit 23 may display a pop-up screen 23a that displays a video acquired by another medical device 32 or the like and processed by the processing device 10.

In addition, the display device 20 includes one or more sensors such as a line-of-sight detection sensor 22a, a sound input sensor 22b, and a behavior (gesture) detection sensor 22c, and also functions as an input interface (also referred to as input device) for the surgical staff to input operation information for a medical device that can be remotely operated on the IP network 60 such as the endoscope system 30. Note that, in the following description, for the sake of clarity, it is assumed that the medical device to be remotely operated is the endoscope system 30.

Although details will be described later, the processing device 10 includes, for example, an information processing apparatus (or system) such as a personal computer, a workstation, a server, or a cloud server, executes predetermined processing on imaging data acquired by the endoscope system 30, the medical device 43, or the like and processed by the processing device 10, and transmits the imaging data after the processing to the display device 20 as display data. In addition, the processing device 10 generates control data for a device that can be remotely operated such as the endoscope system 30 from operation data input from the display device 20, and transmits the generated control data to a target device via the IP network 60.

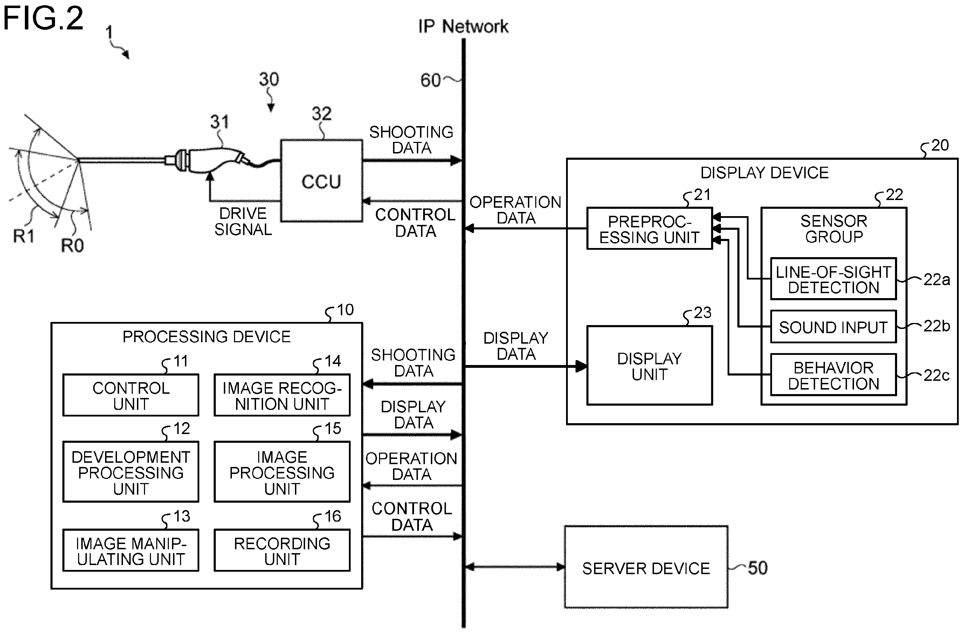

Subsequently, a more detailed configuration of the display device 20 and the processing device 10 according to the present embodiment will be described with reference to FIG. 2. FIG. 2 is a block diagram illustrating a more detailed schematic configuration example of the surgical integrated imaging system according to the present embodiment.

### (Display device 20)

As illustrated in FIG. 2, the display device 20 includes a preprocessing unit 21, a sensor group 22, and the display unit 23.

As described above, the display unit 23 displays the video data (display data) acquired by the endoscope system 30, the medical device 43, or the like and processed by the processing device 10 to a surgical staff such as a primary surgeon, a secondary surgeon, or a nurse. The display unit 23 may be, for example, a display having a resolution of 4K, 8K, or higher.

As described above, the sensor group 22 is an input interface (also referred to as an input device or an input unit) for the surgical staff to input an operation on a medical device that can be remotely operated on the IP network 60 by voice, gesture, or the like. In the present example, as a sensor included in the sensor group 22, a non-contact sensor that acquires information such as line-of-sight, voice, and gesture of the surgical staff, such as the line-of-sight detection sensor 22a, the sound input sensor 22b, and the behavior detection sensor 22c, in a non-contact manner, as described above, is exemplified. However, the present invention is not limited to the non-contact sensor, and a contact sensor or an input device, such as a touch panel, a keyboard, or a mouse, which is directly touched by a surgical staff with a hand or the like, may be used.

For example, the line-of-sight detection sensor 22a may detect a line-of-sight of a specific person such as a primary surgeon, or may detect a line-of-sight of all the surgical staff members. Note that, instead of the line-of-sight detection sensor 22a, a line-of-sight may be detected from an image acquired by a camera. The sound input sensor 22b may be, for example, a microphone or the like. The behavior detection sensor 22c may be, for example, a camera, a distance measurement sensor, or the like.

For example, the preprocessing unit 21 executes recognition processing on sensor information (line-of-sight data, sound data, image data, and the like) input from each sensor of the sensor group 22 to recognize which surgical staff member inputs what operation information to which medical device. In other words, the preprocessing unit 21 functions as an acquisition unit that acquires operation information for remotely operating the medical device on the basis of the operation input via the sensor group 22. For recognition by the preprocessing unit 21, a video (including an image), an object (including an icon and the like), and the like being displayed on the display unit 23 may be used in combination. For example, in a case where the operation information is recognized on the basis of line-of-sight data of the surgical staff, the operation information may be recognized on the basis of which part of the video displayed on the display unit 23 the surgical staff is looking at, which part of the menu or the object displayed on the display unit 23 the surgical staff is looking at, or the like.

Then, the preprocessing unit 21 generates operation data for the medical device to be operated on the IP network 60 on the basis of the recognition result. In other words, the preprocessing unit 21 also functions as a generation unit that generates operation data for remotely operating the medical device using the operation information. At that time, to protect privacy of a surgical staff member who has input the operation information, the preprocessing unit 21 generates operation data in which information for specifying the recognized surgical staff member is concealed, that is, anonymized operation data in which who has input the operation information is concealed. In other words, the preprocessing unit 21 also functions as an anonymization processing unit that deletes the personal information of the surgical staff member from the operation data. For example, in a case where the surgical staff member who has input the operation information is the primary surgeon, the preprocessing unit 21 generates anonymized operation data that does not include personal information such as the name and number of the primary surgeon specified by recognition. However, for example, information such as a position or a role that does not specify an individual, such as "primary surgeon", "secondary surgeon", or "nurse", may be included in the operation data.

The concealed information may include, for example, a face image, an image of an eye for line-of-sight detection, an iris, a raw voice, and the like that identify an individual of the surgical staff. In addition, conversations that are not necessary in the surgery/procedure, eye contact, gender, instruction communication voice and gesture of the surgical staff, and the like may be included.

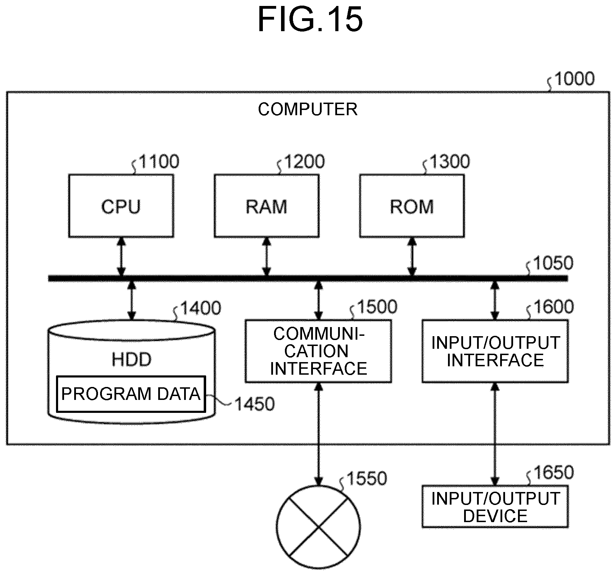

The operation data generated by the preprocessing unit 21 includes at least identification information (device ID) for specifying a medical device to be remotely operated and operation information indicating operation content. The generated operation data is transmitted to the processing device 10 via the IP network 60. This transmission may be performed, for example, a communication interface 150 in FIG. 15. Note that an IP address indicating an address of the medical device to be operated on the IP network 60 may be included instead of the device ID. In addition, identification information (operation ID) associated one-to-one with the operation information or the operation content may be included instead of the operation information. In addition, even in a case where personal information is not included in sensing data (for example, image data) generated by various sensors of the sensor group 22, it is preferable that the operation data does not include sensing data but includes operation information that is a result of analyzing the sensing data. As a result, the data capacity of the information to be output can be reduced.

### (Processing device 10)

The processing device 10 includes, for example, a control unit 11, a development processing unit 12, an image manipulating unit 13, an image recognition unit 14, an image processing unit 15, and a recording unit 16.

The control unit 11 controls each unit of the processing device 10. In addition, the control unit 11 generates control data for controlling a medical device to be operated on the IP network 60 from operation data received via the IP network 60, and transmits the generated control data to a target medical device (for example, endoscope system 30) via the IP network 60.

For example, the development processing unit 12 converts the imaging data received from the CCU 32 of the endoscope system 30 into a data format that can be displayed on the display unit 23.

The image manipulating unit 13 performs manipulating processing such as cutting out a region to be displayed on the display unit 23 and adjustment of the magnification (digital zoom) at the time of display on the display unit 23 on the imaging data converted by the development processing unit 12. The cutout of the region and the adjustment of the magnification on the imaging data may be on the basis of the operation data received via the IP network 60, for example.

The image recognition unit 14 executes recognition processing of an object captured in the imaging data after the conversion by the development processing unit 12 or in the imaging data after the processing by the image manipulating unit 13. For example, in a case where an organ, a blood vessel, or the like of a patient, a surgical instrument such as an electric scalpel or a forceps, a hand of a primary surgeon, a secondary surgeon, or the like is captured in the imaging data, the image recognition unit 14 may specify a position, a region, or the like in the video by recognizing these objects.

The image processing unit 15 generates screen data including the imaging data after the processing by the image manipulating unit 13. This screen data may constitute a user interface for the surgical staff to input operation information by a line-of-sight or the like. The screen data generated by the image processing unit 15 is transmitted to the display device 20 via the IP network 60 as display data to be displayed on the display unit 23, and is displayed on the display unit 23.

For example, the recording unit 16 may accumulate operation data received via the IP network 60 or control data generated by the control unit 11 on the basis of the operation data together with time information in chronological order.

### 1.2 Operation Example

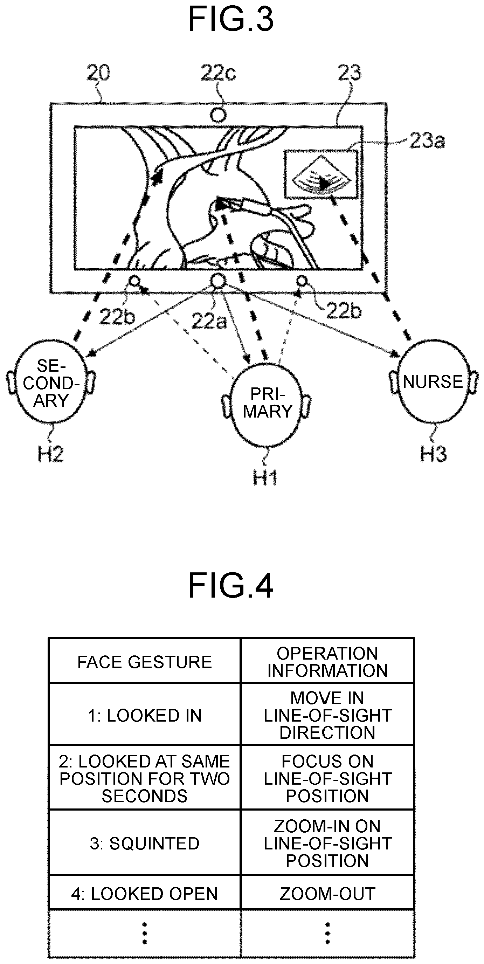

Next, an operation example of the surgical integrated imaging system 1 will be described using a case where the endoscope 31 is remotely operated as an example. FIG. 3 is a diagram illustrating an example of a positional relationship between a display device and a surgical staff according to the present embodiment. As illustrated in FIG. 3, in the following description, there are a primary surgeon H1, a secondary surgeon H2, and a nurse H3 as a surgical staff, and among them, a case will be exemplified where the endoscope 31 is remotely operated on the basis of a gesture with an expression, a behavior, or the like of the face of the primary surgeon H1 (hereinafter, referred to as face gesture), a voice uttered by the primary surgeon H1, or a line-of-sight of the primary surgeon H1.

### 1.2.1 Case of Remote Operation by Face Gesture

First, a case where the endoscope 31 is remotely operated by a face gesture of the primary surgeon H1 will be described. FIG. 4 is a diagram illustrating an example of a correspondence relationship between a face gesture and operation information according to the present embodiment. As illustrated in FIG. 4, in the present description, "1: Looked in", "2: Looked at the same position for two seconds", "3: Squinted", "4: Looked open", ... are exemplified as the face gesture.

For example, "Move in line-of-sight direction" is associated with "1: Looked in" as the operation information. The move in the line-of-sight direction may be, for example, control for shifting the region to be displayed on the display unit 23 in the line-of-sight direction. This control may be realized, for example, by adjusting a region to be cut out from the imaging data on the basis of the operation data by the image manipulating unit 13 of the processing device 10, or may be realized by controlling a robot arm supporting the endoscope 31 to adjust the position and posture of the endoscope 31.

For example, "Focus on line-of-sight position" is associated with "2: Looked at the same position for two seconds" as the operation information. Focusing on the line-of-sight position may be, for example, control to focus the endoscope 31 on an object present ahead of the line-of-sight, among objects, such as an organ or a blood vessel of a patient, a surgical instrument such as an electric scalpel or a forceps, or a hand of a primary surgeon, a secondary surgeon, or the like in a video captured on the display unit 23. This control may be realized, for example, by controlling the focal length of the endoscope 31 so that the focus of the endoscope 31 matches the target object.

For example, "Zoom-in on line-of-sight position" is associated with "3: Squinted" as the operation information. Zooming in on the line-of-sight position may be, for example, control to increase the display magnification of the region of the position in the video captured in the display unit 23. Note that the partial region may be a region that is present ahead of the line-of-sight, the region corresponding to an organ, a blood vessel, or the like of a patient, a surgical instrument such as an electric scalpel or forceps, a hand of a primary surgeon, a secondary surgeon, or the like existing in front of the line-of-sight. This control may be realized, for example, by narrowing down a region to be cut out from imaging data by the image manipulating unit 13 of the processing device 10 on the basis of the operation data and enlarging the cutout video data (digital zoom-in), or may be realized by controlling the lens position of the endoscope 31 to increase the focal length (optical zoom-in) .

For example, "Zoom-out" is associated with "4: Looked open" as the operation information. Zooming out may be, for example, control to increase the display magnification of the video captured in the display unit 23. This control may be realized, for example, by enlarging a region to be cut out from imaging data by the image manipulating unit 13 of the processing device 10 on the basis of the operation data and reducing the cutout video data (digital zoom-out), or may be realized by controlling the lens position of the endoscope 31 to decrease the focal length (optical zoom-out). Alternatively, in a case where digital zoom-in has been executed by the image manipulating unit 13, or in a case where optical zoom-in has been executed by the endoscope 31, these controls may be released to shift to display at the reference magnification.

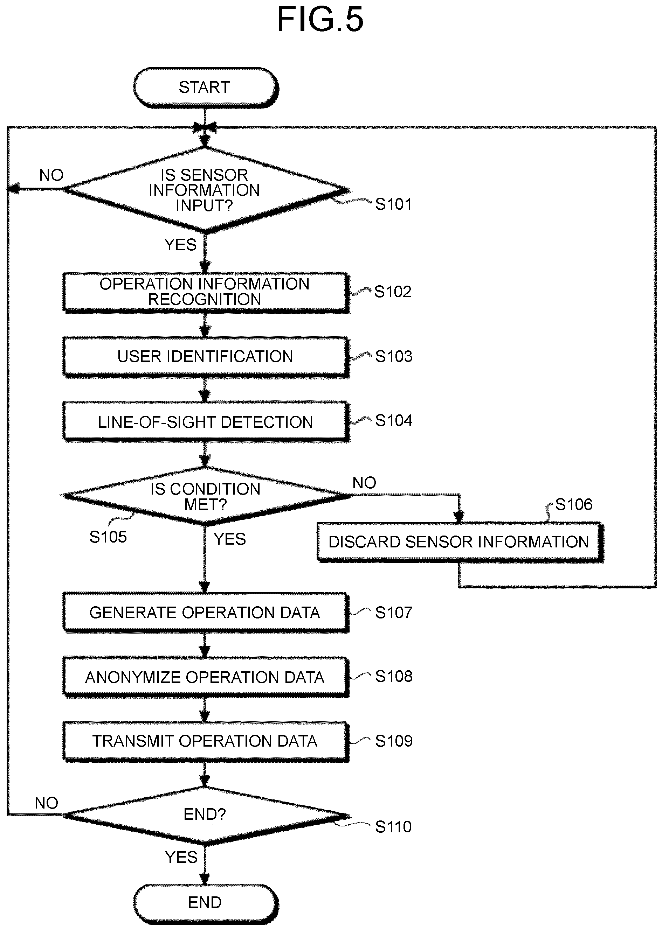

Subsequently, a series of flow of an operation example of remotely operating the endoscope 31 by the face gesture according to the present embodiment will be described with reference to a flowchart illustrated in FIG. 5. Note that, in the following description, attention is paid to the operation of the preprocessing unit 21.

As illustrated in FIG. 5, in a case where the endoscope 31 is remotely operated by the face gesture, first, the preprocessing unit 21 waits until sensor information is input from each sensor of the sensor group 22 (NO in Step S101). The input of the sensor information from the sensor group 22 to the preprocessing unit 21 may be executed at a predetermined period, for example, or may be executed with some signal from the outside as a trigger. In addition, the periods in which the sensors 22a to 22c of the sensor group 22 input the sensor information to the preprocessing unit 21 may be the same or different.

When the sensor information is input from the sensor group 22 (YES in Step S101), the preprocessing unit 21 executes recognition processing on the sensor information input from the behavior detection sensor 22c, for example, to specify a face gesture of one or more surgical staff members included in this sensor information and specify operation information associated with the specified face gesture (Step S102). Note that, in a case where, in the specified face gesture, there is a face gesture that is not associated with operation information, the preprocessing unit 21 may skip specifying operation information related to the face gesture.

In addition, the preprocessing unit 21 executes recognition processing on the sensor information input from the behavior detection sensor 22c, for example, to identify one or more surgical staff members who have performed the face gesture specified in Step S102 (Step S103). Note that the identification processing for a face gesture with which the operation information is not associated may be omitted. In addition, the identification of the surgical staff members may be executed using, for example, image data such as the faces of the surgical staff members registered in advance in the display device 20 or sound data of voice.

Next, the preprocessing unit 21 detects the line-of-sight of each surgical staff member on the basis of, for example, one or more surgical staff members identified in Step S103 and the sensor information input from the line-of-sight detection sensor 22a in Step S101 (Step S104). Note that the line-of-sight detection processing on a face gesture with which the operation information is not associated may be omitted. In addition, in a case where the input of the operation information by the face gesture is permitted only to a specific staff member (in the present description, primary surgeon H1), the preprocessing unit 21 may operate so as to detect the line-of-sight of only the specific staff member in Step S104.

Next, the preprocessing unit 21 determines whether or not the operation information and the surgical staff members specified in Steps S102 and S103, and the line-of-sight of each surgical staff member detected in Step S104 meet a preset condition (Step S105). Here, the condition may be, for example, a condition of whether or not the face gesture is associated with the operation information, whether or not a person who has executed the input of the operation information by the face gesture is a surgical staff member permitted to execute the input (in the present description, primary surgeon H1), or whether or not the line-of-sight faces the display unit 23, or the like.

When the preset condition is not met (NO in Step S105), the preprocessing unit 21 discards the sensor information input in Step S101 and the information specified or detected in Steps S102 to S104 (Step S106), and returns to Step S101.

On the other hand, when the preset condition is met (YES in Step S105), the preprocessing unit 21 generates operation data from the operation information and the surgical staff members specified in Steps S102 and S103 and the line-of-sight detected in Step S104 (Step S107). The operation data to be generated can include the operation information specified in Step S102, the information (personal information) specifying the surgical staff members specified in Step S103, and the information on the line-of-sight detected in Step S104 (hereinafter, referred to as line-of-sight information).

Next, the preprocessing unit 21 anonymizes the operation data by deleting or replacing a part or all of the information (personal information) specifying the surgical staff members from the operation data generated in Step S107 (Step S108).

Subsequently, the preprocessing unit 21 transmits the anonymized operation data to the processing device 10 via the IP network 60 (Step S109). In contrast, the control unit 11 of the processing device 10 generates control data for causing the CCU 32 to control the endoscope 31 from the operation information and the line-of-sight information included in the operation data, and transmits the generated control data to the CCU 32 of the endoscope system 30 via the IP network 60. The CCU 32 generates a drive signal according to the received control data and inputs the drive signal to the endoscope 31. As a result, the endoscope 31 is controlled on the basis of the operation information (and the line-of-sight information) specified from the face gesture.

Thereafter, the preprocessing unit 21 determines whether or not to end the present operation (Step S110), and ends the present operation when it is determined to end the present operation (YES in Step S110). On the other hand, when it is determined not to end the present operation (NO in Step S110), the preprocessing unit 21 returns to Step S101 and executes subsequent operations.

### 1.2.2 Case of Remote Operation on the basis of Line-of-sight

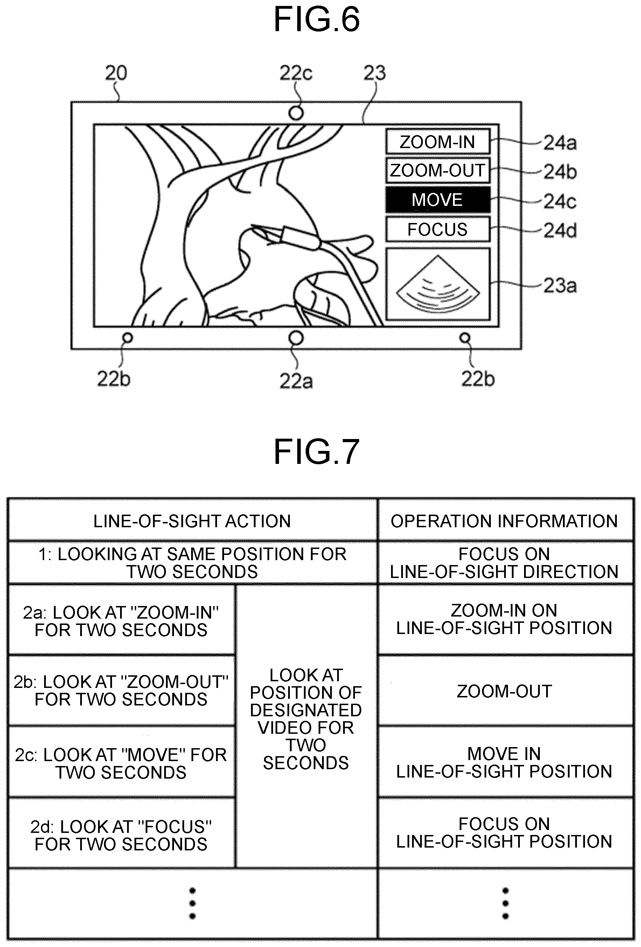

Next, a case where the endoscope 31 is remotely operated on the basis of the line-of-sight of the primary surgeon H1 will be described. FIG. 6 is a diagram illustrating an example of a user interface displayed on the display unit 23 in a case where remote operation of the endoscope 31 is enabled on the basis of the line-of-sight. Note that, in the following description, the movement of the line-of-sight performed by the surgical staff for operation input is referred to as a line-of-sight action. FIG. 7 is a diagram illustrating an example of a correspondence relationship between a line-of-sight action and operation information according to the present embodiment.

As illustrated in FIG. 6, in a case where the remote operation of the endoscope 31 by the line-of-sight action is enabled, the display unit 23 displays menu objects 24a to 24d for receiving the input of the operation information by the line-of-sight action in addition to the video acquired by the endoscope system 30, the medical device 43, or the like and processed by the processing device 10 and the pop-up screen 23a.

"Zoom-in" of the menu object 24a is a graphical user interface (GUI) for inputting operation information of "Zoom-in to line-of-sight position". "Zoom-out" of the menu object 24b is a GUI for inputting operation information of "Zoom-out". "Move" of the menu object 24c is a GUI for inputting operation information of "Move to line-of-sight position". "Focus" of the menu object 24d is a GUI for inputting operation information of the "Focus on line-of-sight position". Note that the content of each piece of operation information may be similar to the content described above with reference to FIG. 4.

In addition, as illustrated in FIG. 7, in the present description, examples of the line-of-sight include "1: Looking at the same position for two seconds", "2a: Look at "Zoom-In" for two seconds", "2b: Look at "Zoom-out" for two seconds", "2c: Look at "Move" for two seconds", "2d: Look at "Focus" for two seconds", and .... In addition, an additional line-of-sight action of "Look at position of designated video for two seconds" is associated with 2a to 2d.

For example, "Focus on line-of-sight position" is associated with "1: Looking at the same position for two seconds" as the operation information. Therefore, when the primary surgeon H1 is looking at the same position of the video captured on the display unit 23 for two seconds, the focal length of the endoscope 31 is controlled so that the focus of the endoscope 31 matches the target object.

For example, "Zoom-in on line-of-sight position" is associated with "2a: Look at "Zoom-in" for two seconds" and "Look at position of designated video for two seconds" as the operation information. Therefore, in a case where the primary surgeon H1 looks at the "Zoom-in" of the menu object 24a for two seconds and then looks at the same position (hereinafter, also referred to as line-of-sight position) of the video captured on the display unit 23 for two seconds, control of zooming in the video to be displayed on the display unit 23 to the line-of-sight position (digital zoom-in or optical zoom-out) is executed.

For example, "Zoom-out" is associated with "2b: Look at "Zoom-out" for two seconds" and "Look at position of designated video for two seconds" as the operation information. Therefore, in a case where the primary surgeon H1 looks at the "Zoom-out" of the menu object 24b for two seconds and then looks at the same position (line-of-sight position) of the video captured on the display unit 23 for two seconds, control of zooming out the video to be displayed on the display unit 23 to the line-of-sight position (digital zoom-out or optical zoom-in) is executed.

For example, "Move to line-of-sight position" is associated with "2c: Look at "Move" for two seconds" and "Look at position of designated video for two seconds" as the operation information. Therefore, in a case where the primary surgeon H1 looks at the "Move" of the menu object 24c for two seconds and then looks at the same position (line-of-sight position) of the video captured on the display unit 23 for two seconds, the region cut out from the imaging data by the image manipulating unit 13 of the processing device 10 is adjusted or the position and posture of the endoscope 31 are adjusted by controlling the robot arm supporting the endoscope 31 so that the video to be displayed on the display unit 23 becomes a video centered on the line-of-sight position.

For example, "Focus on line-of-sight position" is associated with "2d: Look at "Focus" for two seconds" and "Look at position of designated video for two seconds" as the operation information. Therefore, in a case where the primary surgeon H1 looks at the "Zoom-in" of the menu object 24a for two seconds and then looks at the same position (line-of-sight position) of the video captured on the display unit 23 for two seconds, the focal length of the endoscope 31 is controlled so that the focus of the endoscope 31 matches the object corresponding to the line-of-sight position.

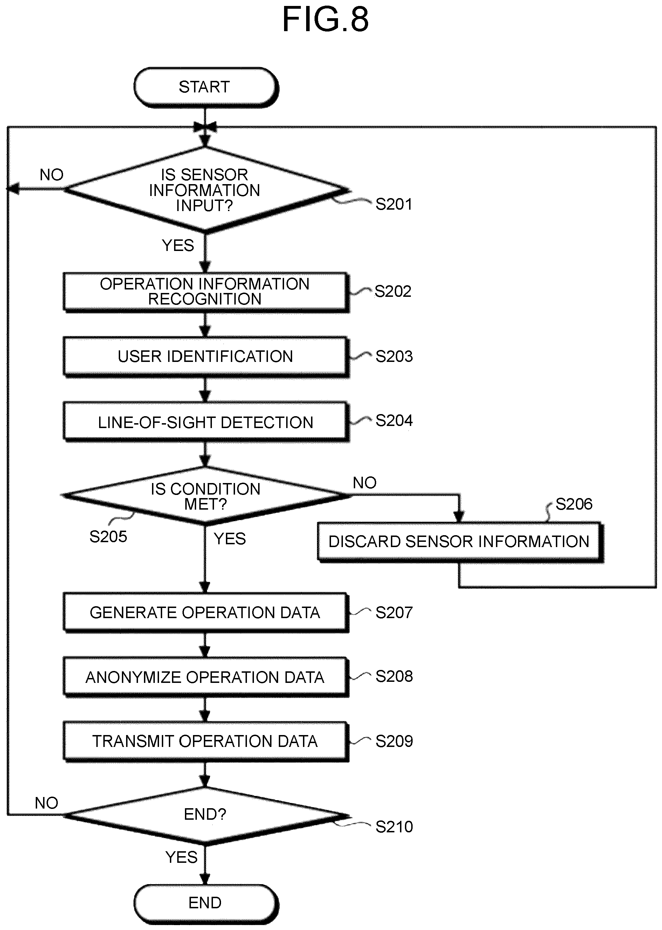

Subsequently, a series of flows in an operation example of remotely operating the endoscope 31 by the line-of-sight action according to the present embodiment will be described with reference to a flowchart illustrated in FIG. 8. Note that, In the following description, attention is paid to the operation of the preprocessing unit 21.

As illustrated in FIG. 8, in a case where the endoscope 31 is remotely operated by the line-of-sight action, first, the preprocessing unit 21 waits until sensor information is input from each sensor of the sensor group 22 as in Step S101 in FIG. 5 (NO in Step S201).

When the sensor information is input from the sensor group 22 (YES in Step S201), the preprocessing unit 21 executes recognition processing on the sensor information input from the line-of-sight detection sensor 22a, for example, to specify operation information associated with the line-of-sight action of each of one or more surgical staff members included in the sensor information (Step S202). Note that, in a case where, in the specified line-of-sight action, there is a line-of-sight action that is not associated with operation information, the preprocessing unit 21 may skip specifying operation information related to the line-of-sight action. In addition, in a case where the line-of-sight action corresponds to any one of 2a to 2d illustrated in FIG. 7, the preprocessing unit 21 may specify, for example, "Set a menu object that has been continuously looked at for two seconds into a selected state" as the operation information. Then, in a case where the line-of-sight action detected in a state where any one of the menu objects 24a to 24d is being selected is "Look at position of designated video for two seconds", the preprocessing unit 21 may specify the operation information corresponding to the menu object (any one of the menu objects 24a to 24d) in the selected state.

In addition, as in Steps S103 and S104 in FIG. 5, the preprocessing unit 21 identifies one or more surgical staff members on the basis of the behavior detection sensor 22c, for example (Step S203), and detects a correspondence relationship between each identified surgical staff member and the detected line-of-sight action (Step S204).

Next, the preprocessing unit 21 determines whether or not the operation information and the surgical staff members specified in Steps S202 and S203, and the line-of-sight action of each surgical staff member detected in Step S204 meet a preset condition (Step S205). Here, the condition may be, for example, a condition of whether or not the line-of-sight action is associated with the operation information, whether or not a person who has executed the input of the operation information by the line-of-sight action is a surgical staff member permitted to execute the input, or whether or not the line-of-sight faces the display unit 23, or the like.

When the preset condition is not met (NO in Step S205), the preprocessing unit 21 discards the sensor information input in Step S201 and the information specified in Steps S202 to S203 (Step S206), and returns to Step S201.

On the other hand, when the preset condition is met (YES in Step S205), the preprocessing unit 21 generates operation data from the operation information and the surgical staff members specified in Steps S202 and S203 (Step S207). The operation data to be generated can include the operation information specified in Step S202, the information (personal information) specifying the surgical staff members specified in Step S203.

Next, the preprocessing unit 21 anonymizes the operation data by deleting or replacing a part or all of the information (personal information) specifying the surgical staff members from the operation data generated in Step S207 (Step S208).

Subsequently, the preprocessing unit 21 transmits the anonymized operation data to the processing device 10 via the IP network 60 (Step S209). In contrast, the control unit 11 of the processing device 10 generates control data for causing the CCU 32 to control the endoscope 31 from the operation information and the line-of-sight information included in the operation data, and transmits the generated control data to the CCU 32 of the endoscope system 30 via the IP network 60. The CCU 32 generates a drive signal according to the received control data and inputs the drive signal to the endoscope 31. As a result, the endoscope 31 is controlled on the basis of the operation information (and the line-of-sight action) specified from the line-of-sight action. However, in a case where the operation information is "Set a menu object that has been continuously looked at for two seconds to a selected state", the image processing unit 15 of the processing device 10 may generate display data (in FIG. 6, as an example, the menu object 24c of "Move" is color-inverted) representing that the menu object (any one of the menu objects 24a to 24d) selected by the line-of-sight action is in the selected state and transmit the display data to the display device 20.

Thereafter, the preprocessing unit 21 determines whether or not to end the present operation (Step S210), and ends the present operation when it is determined to end the present operation (YES in Step S210). On the other hand, when it is determined not to end the present operation (NO in Step S210), the preprocessing unit 21 returns to Step S201 and executes the subsequent operations.

### 1.2.3 Case of Remote Operation by Combination of Line-of-sight and Image Recognition

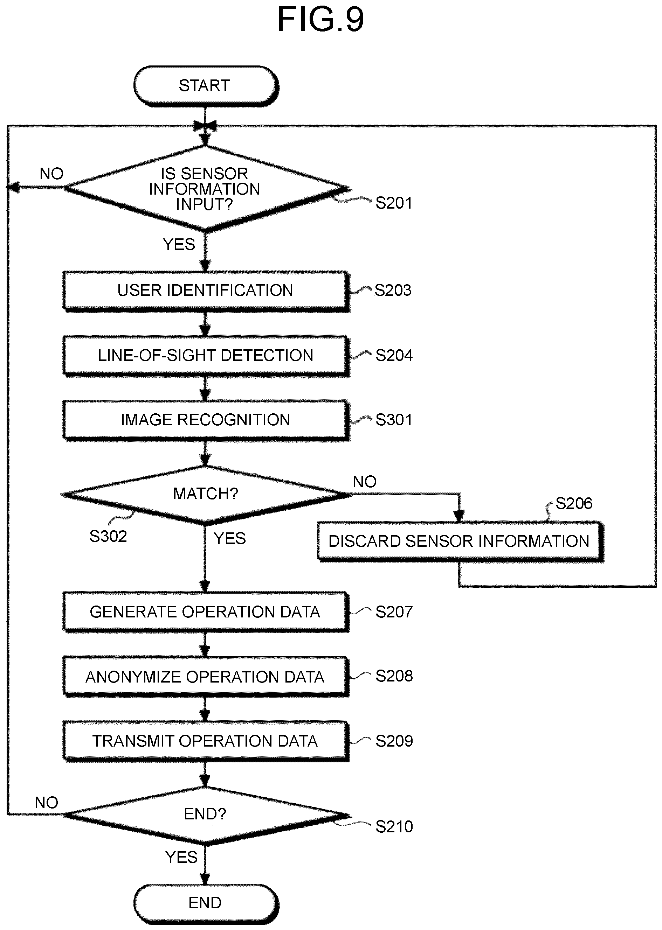

Next, a case where the endoscope 31 is remotely operated by a combination of the line-of-sight of the primary surgeon H1 and image recognition will be described. Note that, in the present description, a case where the focus of the endoscope 31 is remotely operated on the basis of a combination of the line-of-sight of the primary surgeon H1 and image recognition will be exemplified. FIG. 9 is a flowchart illustrating an operation example of remotely operating the focus of the endoscope 31 on the basis of a combination of a line-of-sight and image recognition according to the present embodiment. Note that, In the following description, attention is paid to the operation of the preprocessing unit 21. In addition, in the following description, the same operation as the operation described above with reference to FIG. 8 is cited, and a detailed description thereof will be omitted.

As illustrated in FIG. 9, in a case where the focus of the endoscope 31 is remotely operated on the basis of a combination of the line-of-sight and image recognition, first, the preprocessing unit 21 inputs sensor information from the sensor group 22, identifies the user on the basis of, for example, the sensor information input from the behavior detection sensor 22c, and detects a correspondence relationship between each identified surgical staff member and the detected line-of-sight, as in Steps S201, S203, and S204 in FIG. 8. Note that, in a case where the remote operation is enabled other than the focus of the endoscope 31, as in the flowchart illustrated in FIG. 8, Step S202 may be executed to specify the operation information associated with the line-of-sight action.

Next, the preprocessing unit 21 executes recognition processing on the video data in the display data input from the processing device 10 to specify a position, a region, or the like of a specific object in the video, such as an organ, a blood vessel, or the like of a patient, a surgical instrument such as an electric scalpel or a forceps (or a site such as a tip thereof), or a hand (or a site such as a fingertip) of a primary surgeon, a secondary surgeon, or the like included in the video data (Step S301).

Next, the preprocessing unit 21 determines whether or not the line-of-sight position on the display unit 23 indicated by the line-of-sight of the primary surgeon H1 specified in Step S204 matches the position of the region of the specific object specified in Step S301 on the display unit 23 (Step S302). Note that the recognition processing may be executed, for example, by the image recognition unit 14 of the processing device 10, and the result (information regarding the position, region, and the like of the specific object in the video) may be transmitted to the preprocessing unit 21.

When the line-of-sight position and the position of the region of the specific object do not match (NO in Step S302), that is, when the primary surgeon H1 is not looking at the specific object in the video, the preprocessing unit 21 discards the sensor information input in Step S201 and the information specified in Steps S203 to S204 (Step S206), and returns to Step S201.

On the other hand, when the line-of-sight position and the position of the region of the specific object match (YES in Step S302), that is, when the primary surgeon H1 is looking at the specific object in the video, the preprocessing unit 21 generates operation data from the operation information for focusing on the line-of-sight position indicated by the sensor information (line-of-sight data) input in Step S201 and the information on the surgical staff members specified in Step S203 (Step S207).

Subsequently, as in Steps S208 to S210 in FIG. 8, the preprocessing unit 21 anonymizes the operation data and then transmits the anonymized operation data to the CCU 32 of the endoscope system 30 via the IP network 60. Thereafter, the preprocessing unit 21 determines whether or not to end the present operation (Step S210). When it is determined to end the operation (YES in Step S210), the present operation is ended, and when it is determined not to end the operation (NO in Step S210), the process returns to Step S201, and the subsequent operations are executed.

### 1.2.4 Case of Remote Operation by Combination of Line-of-sight and Voice

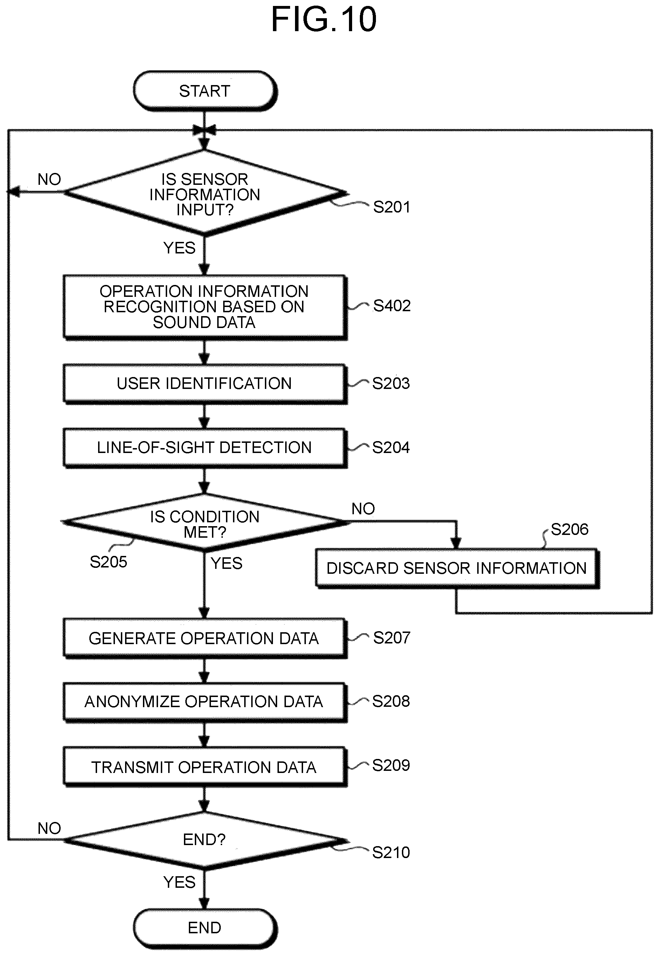

Next, a case where the endoscope 31 is remotely operated by a combination of the line-of-sight and the voice of the primary surgeon H1 will be described. FIG. 10 is a flowchart illustrating an operation example of remotely operating the endoscope 31 on the basis of a combination of a line-of-sight and voice according to the present embodiment. Note that, In the following description, attention is paid to the operation of the preprocessing unit 21. In addition, in the following description, the same operation as the operation described above with reference to FIG. 8 is cited, and a detailed description thereof will be omitted.

As illustrated in FIG. 10, in a case where the endoscope 31 is remotely operated by a combination of a line-of-sight and voice, first, the preprocessing unit 21 waits until sensor information is input from each sensor of the sensor group 22 as in Step S201 in FIG. 8 (NO in Step S201) .

When the sensor information is input from the sensor group 22 (YES in Step S201), the preprocessing unit 21 executes recognition processing on sound data input from the sound input sensor 22b, for example, to specify operation information associated with the utterance content of each of one or more surgical staff members included in the sound data (Step S402). Note that, in a case where, in the specified utterance content, there is an utterance content that is not associated with operation information, the preprocessing unit 21 may skip specifying operation information related to the utterance content.

In addition, as in Steps S203 and S204 in FIG. 8, the preprocessing unit 21 identifies one or more surgical staff members on the basis of the behavior detection sensor 22c, for example, and detects a correspondence relationship between each identified surgical staff member and the detected utterance content.

Next, as in Step S205 in FIG. 8, the preprocessing unit 21 determines whether or not the operation information and the surgical staff members specified in Steps S402 and S203, and the line-of-sight of each surgical staff member detected in Step S204 meet a preset condition, and when the preset condition is not met (NO in Step S205), the preprocessing unit 21 discards the sensor information input in Step S201 and the information specified in Steps S402 to S203 (Step S206), and returns to Step S201.

On the other hand, when the preset condition is met (YES in Step S205), the preprocessing unit 21 generates operation data from the operation information and the surgical staff members specified in Steps S202 and S203 (Step S207).

For example, if the utterance content of the primary surgeon H1 is, for example, "Focus on around here", the preprocessing unit 21 recognizes operation information for controlling the focus of the endoscope 31 in Step S402, and then, generates, in Step S207, operation data for focusing the endoscope 31 on the line-of-sight position detected in Step S204.

In addition, if the utterance content of the primary surgeon H1 is, for example, "Enlarge around here", the preprocessing unit 21 recognizes operation information (digital zoom-in or optical zoom-in) for controlling the magnification of the video to be displayed on the display unit 23 in Step S402, and then, generates, in Step S207, operation data for enlarging the video to be displayed on the display unit 23 (digital zoom-in or optical zoom-in) while setting the line-of-sight position detected in Step S204 as the center of the display unit 23.

Alternatively, if the utterance content of the primary surgeon H1 is, for example, "Enlarge around here", the preprocessing unit 21 recognizes operation information for controlling the magnification of the video to be displayed on the display unit 23 (digital zoom-in or optical zoom-in) in Step S402 and operation information for controlling the focus of the endoscope 31, and then, generates, in Step S207, operation data for enlarging the video to be displayed on the display unit 23 (digital zoom-in or optical zoom-in) while setting the line-of-sight position detected in Step S204 as the center of the display unit 23 and for focusing on the object corresponding to the line-of-sight position.

Note that the operation data to be generated in Step S207 can include the operation information specified in Step S202, the information (personal information) specifying the surgical staff members specified in Step S203.

Thereafter, the preprocessing unit 21 executes the same operations as Steps S208 to S210 in FIG. 8 to transmit anonymized operation data to the processing device 10 via the IP network 60.

### 1.2.5 Case of Remote Operation with Specific Operation Sound as Trigger

Next, an example of a case where a medical device such as the endoscope 31 is remotely operated by the use of a specific surgical instrument such as an electric scalpel or an aspirator as a trigger will be described.

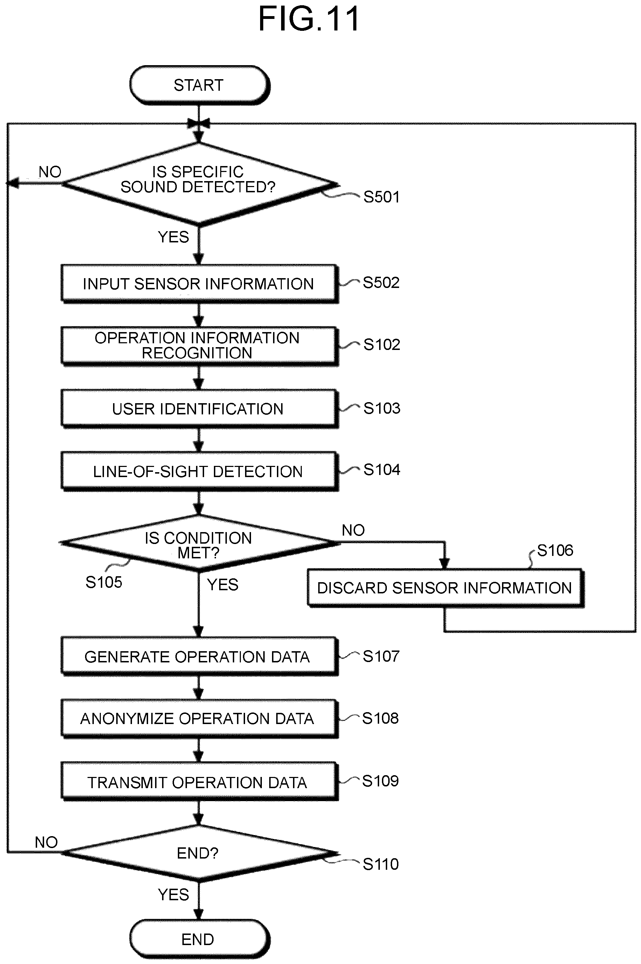

FIG. 11 is a flowchart illustrating an operation example of remotely operating the endoscope 31 with a specific operation sound as a trigger according to the present embodiment. Note that, In the following description, attention is paid to the operation of the preprocessing unit 21. In addition, in the following description, the same operation as the operation described above with reference to FIG. 5 is cited, and a detailed description thereof will be omitted.

As illustrated in FIG. 11, in a case where the endoscope 31 is remotely operated with a specific operation sound emitted by a specific surgical instrument such as an electric scalpel or an aspirator as a trigger, first, the preprocessing unit 21 recognizes sound data input from the sound input sensor 22b, thereby monitoring whether or not a specific operation sound (hereinafter, referred to as specific sound) that is emitted when the specific surgical instrument is used is detected (Step S501). Note that waveform data and the like related to the specific sound may be stored in a storage area in the display device 20 in advance.

When the specific sound is detected (YES in Step S501), the preprocessing unit 21 inputs sensor information from each sensor of the sensor group 22 (Step S502). Thereafter, the preprocessing unit 21 executes the same operations as Steps S102 to S110 in FIG. 5 to transmits anonymized operation data to the processing device 10 via the IP network 60.

Note that, in the present description, the case where the operation example illustrated in FIG. 5 is used as a base is exemplified, but the present invention is not limited thereto, and other operation examples illustrated in FIGS. 8 to 10 may be used as a base.

In addition, in the present description, the case where the operation data based on the sensor information input in Step S502 is generated in Step S107 has been exemplified, but the present invention is not limited thereto. For example, in Step S102, operation information for a case where the specific sound is detected may be determined in advance, and in a case where the specific sound is detected in Step S501, the operation data may be generated on the basis of the operation information determined in advance in Step S107. For example, in a case where the operating sound of the electric scalpel is detected in Step S501, instead of Step S102, operation information for instructing focusing of the endoscope 31 may be determined in advance, and operation data for focusing of the endoscope 31 on the line-of-sight position detected in Step S104 may be generated in Step S107.

### 1.2.6 Case of Remote Operation of Other Electronic Device

Note that, in the above description, the case where the medical device connected to the IP network 60 is remotely operated has been exemplified, but the target of the remote operation is not limited to the medical device. For example, in a case where the surgeon listens to music to calm down during surgery, a music player may be the target of the remote operation. Note that the music player may be an electronic device connected to the IP network 60, an application installed in the server device 50 or the like, a music distribution service using an external cloud, or the like. In addition, the present invention is not limited to the music player, and for example, various electronic devices and applications such as a video player that reproduces a past surgical moving image or the like performed by a surgical method similar to the surgical method of the current surgery, and an application for a meeting used for communication with staff members outside the operating room may be the target of the remote operation.

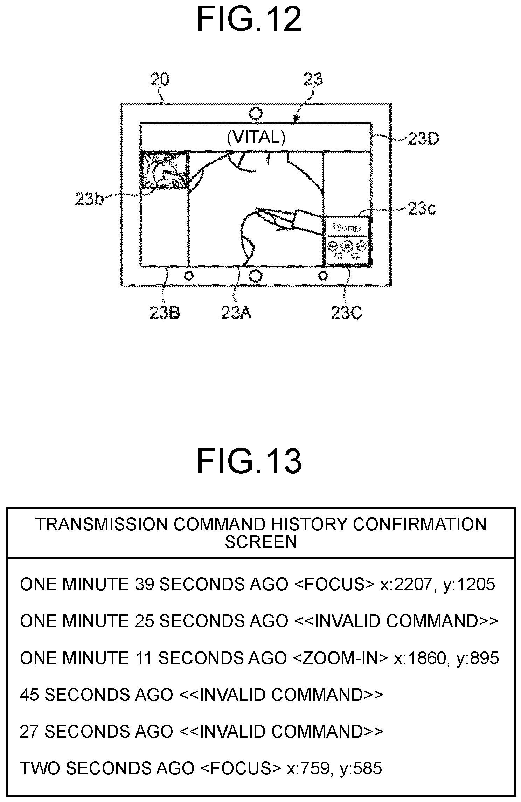

FIG. 12 is a diagram illustrating an example of a screen displayed on the display unit according to the present embodiment. As illustrated in FIG. 12, in the present example, the display unit 23 is divided into a first display region 23A, a second display region 23B, a third display region 23C, and a fourth display region 23D.

The first display region 23A has, for example, a larger display area than other display regions, and is located substantially at the center of the display unit 23. In the first display region 23A, for example, an enlarged video of a region on which the primary surgeon H1 or the like is focusing during surgery may be displayed.

For example, the second display region 23B is located on one side (left side in FIG. 12) of the first display region 23A. In the second display region 23B, for example, a whole video 23b acquired by the endoscope 31 may be displayed. In addition, the enlarged video displayed in the first display region 23A may be an enlarged video of a region on which the primary surgeon H1 focuses with respect to the whole video 23b of the second display region 23B. Any one of the operations illustrated in FIGS. 5 and 8 to 11 described above may be used to input the focused region.

For example, the third display region 23C is located on the other side (right side in FIG. 12) of the first display region 23A. The third display region 23C may be used for multiple purposes. Therefore, in the present example, a GUI 23c for remotely operating the music player is displayed in the third display region 23C.

The fourth display region 23D is located, for example, above or below the first display region 23A to the third display region 23C. In the fourth display region 23D, for example, a patient's vital measured during surgery may be displayed.

The surgical staff such as the primary surgeon H1 remotely operates the music player using the GUI 23c according to any one of the operation examples described above with reference to FIGS. 5 and 8 to 11, for example. For example, the primary surgeon H1 may cause the music player to play desired music by fixing the line-of-sight to a title icon ("Song"), a stop icon, a cue icon, a repeat icon, or the like in the GUI 23c for a certain period of time. Alternatively, the primary surgeon H1 may cause the music player to play desired music by speaking "Start playing music" or "Play "Song"" with turning the line-of-sight to any one of the icons. Note that, although not illustrated in FIG. 12, an icon for operating volume-up or volume-down may be displayed.

In addition, the display of the GUI 23c on the third display region 23C may be instructed by the primary surgeon H1 with voice, line-of-sight, or the like, for example. Furthermore, the display of the GUI 23c may be hidden after playing music is started. Furthermore, in a case where an alert, a warning sound, or the like of the device is detected during the surgery, the preprocessing unit 21 may automatically generate an instruction to stop playing music and transmit the instruction to the music player.

### 1.3 Display of Transmission Command History

Note that the contents of the operation data transmitted from the display device 20 to the processing device 10, that is, the operation data transmitted from the display device 20 to the IP network 60 may be displayed on the display unit 23 in chronological order for confirmation by the surgical staff as illustrated in FIG. 13. Note that the command history confirmation screen illustrated in FIG. 13 may be displayed, for example, in the third display region 23C in FIG. 12. The surgical staff can confirm that information regarding privacy is not output to the outside by making it possible to confirm the content of the operation data transmitted from the display device 20 to the IP network 60, so that it is possible to use the surgical integrated imaging system 1 with a sense of security.

### 1.4 Modification of System Configuration

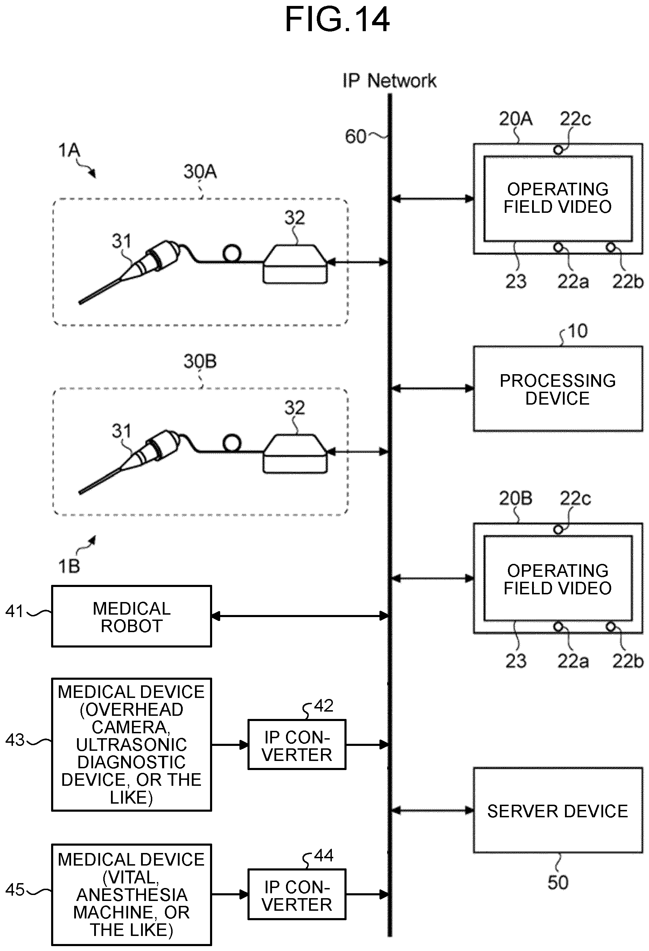

FIG. 1 illustrates a case where one surgical integrated imaging system 1 is connected on the IP network 60, that is, a case where the processing device 10 relays one set of medical device (endoscope system 30) and the display device 20, but the present invention is not limited thereto. For example, as illustrated in FIG. 14, two or more surgical integrated imaging systems 1A and 1B may be connected on the IP network 60, and one processing device 10 may be shared by the respective surgical integrated imaging systems 1A and 1B. In that case, the surgical integrated imaging system 1A includes the processing device 10, a display device 20A, and an endoscope system 30A, and the surgical integrated imaging system 1A includes the processing device 10, a display device 20B, and an endoscope system 30B. In addition, the surgical integrated imaging systems 1A and 1B may be installed in different operating rooms. In that case, the processing device 10 may be installed in any one of the operating rooms, or may be installed in a server room or the like different from the operating room.

### 1.5 Summary

As described above, according to the present embodiment, even in a case where a medical device that can be remotely operated and an input device for remotely operating the medical device are connected via an unclosed network such as the IP network 60, operation data output from the input device can be operation data that have been anonymized, so that it is possible to prevent information regarding privacy of a staff member who operates the medical device and a patient from leaking to the outside. As a result, it is possible to improve the protection of privacy of a person who is involved in surgery or diagnosis.

### 2. Hardware Configuration

The processing device 10 and the display device 20 (for example, the preprocessing unit 21) according to the above-described embodiment and the modifications thereof can be implemented by a computer 1000 having a configuration as illustrated in FIG. 15, for example. FIG. 15 is a hardware configuration diagram illustrating an example of the computer 1000 that implements each function of the processing device 10 and the display device 20 (for example, the preprocessing unit 21). The computer 1000 includes a CPU 1100, a RAM 1200, a read only memory (ROM) 1300, a hard disk drive (HDD) 1400, a communication interface 1500, and an input/output interface 1600. Each unit of the computer 1000 is connected by a bus 1050.

The CPU 1100 operates on the basis of a program stored in the ROM 1300 or the HDD 1400, and controls each unit. For example, the CPU 1100 develops a program stored in the ROM 1300 or the HDD 1400 in the R_AM 1200, and executes processing corresponding to various programs.

The ROM 1300 stores a boot program such as a basic input output system (BIOS) to be executed by the CPU 1100 when the computer 1000 is activated, a program depending on the hardware of the computer 1000, and the like.

The HDD 1400 is a computer-readable recording medium in which a program executed by the CPU 1100, data used by the program, and the like are non-transiently recorded. Specifically, the HDD 1400 is a recording medium in which a projection control program according to the present disclosure as an example of program data 1450 is recorded.

The communication interface 1500 is an interface for the computer 1000 to connect to an external network 1550 (for example, the Internet). For example, the CPU 1100 receives data from another device or transmits data generated by the CPU 1100 to another device via the communication interface 1500.

The input/output interface 1600 has a configuration including the I/F unit 18 described above, and is an interface for connecting an input/output device 1650 and the computer 1000. For example, the CPU 1100 receives data from an input device such as a keyboard and a mouse via the input/output interface 1600. In addition, the CPU 1100 transmits data to an output device such as a display, a speaker, or a printer via the input/output interface 1600. In addition, the input/output interface 1600 may function as a media interface that reads a program or the like recorded in a predetermined recording medium (medium). The medium is, for example, an optical recording medium such as a digital versatile disc (DVD) or a phase change rewritable disk (PD), a magneto-optical recording medium such as a magneto-optical disk (MO), a tape medium, a magnetic recording medium, a semiconductor memory, or the like.

For example, in a case where the computer 1000 functions as each of the processing device 10 and the display device 20 (for example, the preprocessing unit 21) according to the above-described embodiment, the CPU 1100 of the computer 1000 executes a program loaded on the RAM 1200 to implement each function of the processing device 10 and the display device 20 (for example, the preprocessing unit 21). In addition, a program and the like according to the present disclosure are stored in the HDD 1400. Note that the CPU 1100 reads the program data 1450 from the HDD 1400 and executes the program data, but as another example, these programs may be acquired from another device via the external network 1550.

Although the preferred embodiments of the present disclosure have been described in detail with reference to the accompanying drawings, the technical scope of the present disclosure is not limited to such examples. It is obvious that a person having ordinary knowledge in the technical field of the present disclosure can conceive various changes or modifications within the scope of the technical idea described in the claims, and it is naturally understood that these also belong to the technical scope of the present disclosure.

In addition, the effects described in the present specification are merely illustrative or exemplary, and are not restrictive. That is, the technology according to the present disclosure can exhibit other effects obvious to those skilled in the art from the description of the present specification together with or instead of the above effects.

For example, although the embodiment disclosed in the present specification aims to increase confidentiality, it is understood that a configuration in which not sensing data (for example, image data) that is data output from a sensor but operation information that is a result of analyzing the sensing data is output for the purpose of reducing the data capacity of the operation data belongs to the technical scope of the present disclosure.

Note that the following configurations also belong to the technical scope of the present disclosure.
(1) An information processing apparatus including:
   an input unit configured to acquire information including an operation by a user on a medical device on a computer network;
   an acquisition unit configured to acquire operation information for remotely operating the medical device on a basis of the operation input to the input unit;
   a generation unit configured to generate operation data for remotely operating the medical device using the operation information; and
   a transmission unit configured to transmit the operation data to the computer network,
   wherein the operation data does not include personal information of the user.
(2) The information processing apparatus according to (1),
   wherein the input unit includes a non-contact sensor that inputs the operation by the user in a non-contact manner.
(3) The information processing apparatus according to (1) or (2),
   wherein the input unit includes at least one of a line-of-sight detection sensor that detects a line-of-sight of the user, a sound input sensor that inputs a voice uttered by the user, and a behavior detection sensor that detects a behavior of the user, and
   the acquisition unit acquires the operation information on a basis of at least one of line-of-sight data input from the line-of-sight detection sensor, sound data input from the sound input sensor, and image data input from the behavior detection sensor.
(4) The information processing apparatus according to (3),
   wherein the acquisition unit acquires the operation information on a basis of a behavior of a line-of-sight of the user detected by the line-of-sight detection sensor.
(5) The information processing apparatus according to (3) or (4),
   wherein the acquisition unit acquires the operation information on a basis of a voice of the user detected by the sound input sensor.
(6) The information processing apparatus according to any one of (3) to (5),
   wherein the acquisition unit acquires the operation information on a basis of an operation sound of a specific surgical instrument detected by the sound input sensor.
(7) The information processing apparatus according to any one of (3) to (6),
   wherein the acquisition unit acquires the operation information on a basis of a behavior of the user detected by the behavior detection sensor.
(8) The information processing apparatus according to any one of (3) to (7), further including
   a display unit configured to display a video,
   wherein the acquisition unit specifies a position being looked at by the user in the video displayed on the display unit on a basis of the line-of-sight of the user detected by the line-of-sight detection sensor, and acquires the operation information on a basis of the position being looked at by the user in the video.
(9) The information processing apparatus according to (8),
   wherein the video is a video acquired by the medical device.
(10) The information processing apparatus according to (9),
   wherein the display unit further displays an operation menu for the medical device, and
   the acquisition unit specifies a position being looked at by the user in the operation menu displayed on the display unit on a basis of the line-of-sight of the user detected by the line-of-sight detection sensor, and acquires the operation information on a basis of the position being looked at by the user in the video.
(11) The information processing apparatus according to any one of (1) to (10),
   wherein the acquisition unit specifies the user who has input the operation on a basis of the information acquired by the input unit, and acquires the operation information in a case where the specified user is a user who is permitted to perform remote operation on the medical device in advance, and
   the generation unit generates the operation data including the personal information of the user specified by the acquisition unit.
(12) The information processing apparatus according to (11),
   wherein the acquisition unit discards the operation input to the input unit in a case where the specified user is not a user who is permitted to perform remote operation on the medical device in advance.
(13) The information processing apparatus according to any one of (1) to (12),
   wherein the personal information includes at least one of a face image, an eye image, an iris, and a voice of the user.
(14) The information processing apparatus according to any one of (1) to (13),
   wherein the medical device is an endoscope.
(15) An information processing system including:
   a medical device connected to a computer network;
   an input device configured to input an operation for remotely operating the medical device via the computer network; and
   a processing device configured to generate control data for controlling the medical device on a basis of the operation input to the input device,
   wherein the input device includes:
      an input unit configured to acquire information including an operation by a user on the medical device;
      an acquisition unit configured to acquire operation information for remotely operating the medical device on a basis of the operation input to the input unit;
      a generation unit configured to generate operation data for remotely operating the medical device using the operation information; and
      a transmission unit configured to transmit the operation data to the computer network,
      the processing device includes a control unit configured to generate the control data on a basis of the operation data received via the computer network, and
      the operation data does not include personal information of the user.
(16) The information processing system according to (15),
   wherein the input device includes a display unit,
   the medical device includes an endoscope and a control device configured to transmit imaging data acquired by the endoscope via the computer network, and
   the processing device includes an image processing unit configured to generate display data to be displayed on the display unit by using the imaging data received via the computer network, and a transmission unit configured to transmit the display data generated by the image processing unit to the input device via the computer network.
(17) An information processing method including:
   acquiring information including an operation by a user on a medical device on a computer network;
   acquiring operation information for remotely operating the medical device on a basis of the operation input to the input unit;
   generating operation data for remotely operating the medical device using the operation information; and
   transmitting the operation data to the computer network,
   wherein the operation data does not include personal information of the user.
(18) A program for causing a computer for remotely operating a medical device on a computer network to function, the program causing the computer to execute:
   a step of acquiring information including an operation by a user on the medical device on a computer network;
   a step of acquiring operation information for remotely operating the medical device on a basis of the operation input to the input unit;
   a step of generating operation data for remotely operating the medical device using the operation information; and
   a step of transmitting the operation data to the computer network,
   wherein the operation data does not include personal information of the user.
(19) An information processing apparatus including:
   an input unit configured to acquire an operation instruction by a user on a medical device on a computer network;
   an acquisition unit configured to acquire operation information for operating the medical device on a basis of the operation instruction input to the input unit; and
   an output unit configured to output the operation information to a device on the computer network,
   wherein the operation information does not include personal information of the user included in operation data of the user.

### Reference Signs List

- 1, 1A, 1B: SURGICAL INTEGRATED IMAGING SYSTEM
- 10: PROCESSING DEVICE
- 11: CONTROL UNIT
- 12: DEVELOPMENT PROCESSING UNIT
- 13: IMAGE MANIPULATING UNIT
- 14: IMAGE RECOGNITION UNIT
- 15: IMAGE PROCESSING UNIT
- 16: RECORDING UNIT
- 20, 20A, 20B: DISPLAY DEVICE
- 21: PREPROCESSING UNIT
- 22: SENSOR GROUP
- 22a: LINE-OF-SIGHT DETECTION SENSOR
- 22b: SOUND INPUT SENSOR
- 22c: BEHAVIOR DETECTION SENSOR
- 23: DISPLAY UNIT
- 23A: FIRST DISPLAY REGION
- 23B: SECOND DISPLAY REGION
- 23C: THIRD DISPLAY REGION
- 23D: FOURTH DISPLAY REGION
- 23a: POP-UP SCREEN
- 23b: WHOLE VIDEO
- 23c: GUI
- 24a to 24d: MENU OBJECT
- 30, 30A, 30B: ENDOSCOPE SYSTEM
- 31: ENDOSCOPE
- 32: CCU
- 41: MEDICAL ROBOT
- 42, 44: IP CONVERTER
- 43, 45: MEDICAL DEVICE
- 50: SERVER DEVICE

## Claims

1. An information processing apparatus including:
an input unit configured to acquire information including an operation by a user on a medical device on a computer network;
an acquisition unit configured to acquire operation information for remotely operating the medical device on a basis of the operation input to the input unit;
a generation unit configured to generate operation data for remotely operating the medical device using the operation information; and
a transmission unit configured to transmit the operation data to the computer network,
wherein the operation data does not include personal information of the user.

2. The information processing apparatus according to claim 1,
wherein the input unit includes a non-contact sensor that inputs the operation by the user in a non-contact manner.

3. The information processing apparatus according to claim 1,
wherein the input unit includes at least one of a line-of-sight detection sensor that detects a line-of-sight of the user, a sound input sensor that inputs a voice uttered by the user, and a behavior detection sensor that detects a behavior of the user, and
the acquisition unit acquires the operation information on a basis of at least one of line-of-sight data input from the line-of-sight detection sensor, sound data input from the sound input sensor, and image data input from the behavior detection sensor.

4. The information processing apparatus according to claim 3,
wherein the acquisition unit acquires the operation information on a basis of a behavior of a line-of-sight of the user detected by the line-of-sight detection sensor.

5. The information processing apparatus according to claim 3,
wherein the acquisition unit acquires the operation information on a basis of a voice of the user detected by the sound input sensor.

6. The information processing apparatus according to claim 3,
wherein the acquisition unit acquires the operation information on a basis of an operation sound of a specific surgical instrument detected by the sound input sensor.

7. The information processing apparatus according to claim 3,
wherein the acquisition unit acquires the operation information on a basis of a behavior of the user detected by the behavior detection sensor.

8. The information processing apparatus according to claim 3, further including
a display unit configured to display a video,
wherein the acquisition unit specifies a position being looked at by the user in the video displayed on the display unit on a basis of the line-of-sight of the user detected by the line-of-sight detection sensor, and acquires the operation information on a basis of the position being looked at by the user in the video.

9. The information processing apparatus according to claim 8,
wherein the video is a video acquired by the medical device.

10. The information processing apparatus according to claim 9,
wherein the display unit further displays an operation menu for the medical device, and
the acquisition unit specifies a position being looked at by the user in the operation menu displayed on the display unit on a basis of the line-of-sight of the user detected by the line-of-sight detection sensor, and acquires the operation information on a basis of the position being looked at by the user in the video.

11. The information processing apparatus according to claim 1,
wherein the acquisition unit specifies the user who has input the operation on a basis of the information acquired by the input unit, and acquires the operation information in a case where the specified user is a user who is permitted to perform remote operation on the medical device in advance, and
the generation unit generates the operation data including the personal information of the user specified by the acquisition unit.

12. The information processing apparatus according to claim 11,
wherein the acquisition unit discards the operation input to the input unit in a case where the specified user is not a user who is permitted to perform remote operation on the medical device in advance.

13. The information processing apparatus according to claim 1,
wherein the personal information includes at least one of a face image, an eye image, an iris, and a voice of the user.

14. The information processing apparatus according to claim 1,
wherein the medical device is an endoscope.

15. An information processing system including:
a medical device connected to a computer network;
an input device configured to input an operation for remotely operating the medical device via the computer network; and
a processing device configured to generate control data for controlling the medical device on a basis of the operation input to the input device,
wherein the input device includes:
an input unit configured to acquire information including an operation by a user on the medical device;
an acquisition unit configured to acquire operation information for remotely operating the medical device on a basis of the operation input to the input unit;
a generation unit configured to generate operation data for remotely operating the medical device using the operation information; and
a transmission unit configured to transmit the operation data to the computer network,
the processing device includes a control unit configured to generate the control data on a basis of the operation data received via the computer network, and
the operation data does not include personal information of the user.

16. The information processing system according to claim 15,
wherein the input device includes a display unit,
the medical device includes an endoscope and a control device configured to transmit imaging data acquired by the endoscope via the computer network, and
the processing device includes an image processing unit configured to generate display data to be displayed on the display unit by using the imaging data received via the computer network, and a transmission unit configured to transmit the display data generated by the image processing unit to the input device via the computer network.

17. An information processing method including:
acquiring information including an operation by a user on a medical device on a computer network;
acquiring operation information for remotely operating the medical device on a basis of the operation input to the input unit;
generating operation data for remotely operating the medical device using the operation information; and
transmitting the operation data to the computer network,
wherein the operation data does not include personal information of the user.

18. A program for causing a computer for remotely operating a medical device on a computer network to function, the program causing the computer to execute:
a step of acquiring information including an operation by a user on the medical device on a computer network;
a step of acquiring operation information for remotely operating the medical device on a basis of the operation input to the input unit;
a step of generating operation data for remotely operating the medical device using the operation information; and
a step of transmitting the operation data to the computer network,
wherein the operation data does not include personal information of the user.

19. An information processing apparatus including:
an input unit configured to acquire an operation instruction by a user on a medical device on a computer network;
an acquisition unit configured to acquire operation information for operating the medical device on a basis of the operation instruction input to the input unit; and
an output unit configured to output the operation information to a device on the computer network,
wherein the operation information does not include personal information of the user included in operation data of the user.
